# EUROPEAN PATENT SPECIFICATION

(11) **EP 3 903 808 B1**
(45) Date of publication and mention of the grant of the patent: **30.07.2025**
(21) Application number: 20815595.2
(22) Date of filing: 28.05.2020
(51) Int. Cl.: A61L 26/00, A61L 24/00, A61L 24/10, A61K 38/48, A61K 9/16, A61P 7/04, A61K 38/39

(54) **HEMOSTATIC COMPOSITION AND RECEPTACLE COMPRISING SAME**
HÄMOSTATISCHE ZUSAMMENSETZUNG UND BEHÄLTER DAMIT
COMPOSITION HÉMOSTATIQUE ET CONTENANT ASSOCIÉ

(30) Priority: 28.05.2019 KR 20190062613
(43) Date of publication of application: 03.11.2021
(73) Proprietor: Dalim Tissen Co., Ltd., Seoul 03982 (KR)
(72) Inventor: PARK, Si Nae, Seoul 03721 (KR); BAE, Sang Hee, Suwon-si, Gyeonggi-do 16671 (KR); KO, Jae Hyung, Seoul 01010 (KR)
(74) Representative: Petraz, Davide Luigi
(86) International application number: PCT/KR2020/006945
(87) International publication number: WO 2020/242231

(56) References cited:
- EP-A1- 0 171 352
- EP-A1- 2 575 775
- EP-A2- 3 718 578
- WO-A2-2019/107887
- KR-A- 20050 100 626
- KR-A- 20100 048 751
- KR-A- 20130 055 847
- KR-B1- 101 814 841
- US-A1- 2005 284 809
- US-A1- 2012 021 058

## Description

### TECHNICAL FIELD

The present application claims priority based on Korean Patent Application No. 10-2019-0062613, filed on May 28, 2019.

The present invention relates to a hemostatic composition and a container holding the hemostatic composition.

### BACKGROUND ART

Research and development in the field of tissue adhesives containing sealants and hemostats is in a rapid progress. A fibrin sealant was approved by the Food and Drug Administration (FDA) of the United States in 1998, and since then, new tissue adhesives have been continuously developed every year. These tissue adhesives are in the limelight as materials that can substitute for techniques such as suturing, clipping, and cautery, which have been traditionally used in surgical and medical operations.

Although existing surgical techniques such as suturing exhibit strong tensile strength, those surgical techniques have problems such as the pain felt by patients and the necessity of removal after surgical procedures. On the other hand, tissue adhesives have numerous advantages such as a rapid adhesion time, convenient usability, and no necessity of removal after procedures; however, tissue adhesives have limitations such as low adhesiveness, low tensile strength, and noticeable deterioration of adhesiveness in the presence of moisture. Studies for overcoming such limitations of the tissue adhesives are still in progress.

Since tissue adhesives are brought into direct contact with tissues, tissue adhesives are required to have biocompatibility. Furthermore, in the case of medical adhesives, since they are conventionally used in living bodies, when the adhesive substantially flows into the body fluid and blood, the living body is directly involved, and accordingly, there is a demand for a material having stricter biocompatibility and biodegradability, which exhibits reduced toxicity and harmfulness under more stringent conditions.

The tissue adhesives that have been currently commercialized and/or practicalized are roughly classified into cyanoacrylate instant adhesives, fibrin glues, gelatin glues, and polyurethane-based adhesives. Recently, attention has been paid to cyanoacrylate instant adhesives in the studies for instant adhesives having high functionality and high performance. Especially, medical instant adhesives for biological tissue suturing with biocompatibility, flexibility, and low toxicity not only have a hemostatic effect and an antibacterial effect but also can replace sutures, and therefore, research has been actively carried out in developed countries.

These cyanoacrylate-based tissue adhesives are currently commercially available as manufactured products such as Dermabond (Johnson & Johnson) and Indermil (US Surgical). Such a cyanoacrylate-based tissue adhesive is a single substance and has major advantages such as being curable by moisture without using an initiator at room temperature in a short time period and having transparent external appearance and high adhesive strength; however, the tissue adhesive has a disadvantage of being vulnerable to impact and having poor heat resistance. Furthermore, cyanoacrylate-based tissue adhesives are so toxic that they are hardly used at the present time, and although those cyanoacrylate-based tissue adhesives are partially used clinically in countries other than the United States, use of those tissue adhesives has been limited due to histotoxicity and vulnerability.

Furthermore, regarding fibrin glue adhesives, application thereof to cardiosurgery was approved for the first time in 1988 by the FDA in the United States. Thereafter, research and development has been actively conducted on fibrin tissue adhesives, and currently manufactured products such as Tisseel VH (Baxer) and Evicel TM (Johnson & Johnson) are commercially available. Fibrin-based tissue adhesives occupy a major portion of the tissue adhesive market together with cyanoacrylate-based adhesives. The fibrin tissue adhesives utilize a crosslinking reaction of fibrin, and clinical application thereof for replacement or reinforcement of sutures has been implemented by applying fibrinogen, thrombin, calcium chloride, and an inhibitor of fibrinolysin as a tissue adhesive to suturing of peripheral nerves, suturing of microvessels, and the like. Such fibrin glue adhesives have excellent advantages that the fibrin glue adhesives are not affected by moisture at the site of adhesion, adhere rapidly, do not exhibit coagulation disorder with platelets, and have excellent biocompatibility. However, those fibrin glue adhesives have limitations such as weak adhesive force, a rapid rate of biodegradation, and a risk of blood infection.

Furthermore, gelatin glues are biologically derived tissue adhesives, and gelatin glues obtained by crosslinking a gelatin-resorcinol-formalin (GRF) system have been developed. In addition to that, tissue adhesives such as gelatin-glutaraldehyde have been developed, which exhibit high tissue adhesiveness; however, those tissue adhesives have a disadvantage that formalin or glutaraldehyde, both of which are used as crosslinking agents, undergo a crosslinking reaction also with proteins in the biological body and cause histotoxicity.

Furthermore, polyurethane-based adhesives have been developed in the form of elastic adhesives in which the flexibility of the joined part is maintained after curing. These adhesives have a feature that the adhesives absorb water on the surface of biological tissue and increase the adhesiveness to the tissue and react with water to cure within several minutes, and that a cured product of the adhesive is flexible, and there is an advantage that a cured adhesive is appropriately biodegraded. However, these adhesives have a disadvantage that aromatic diisocyanates, which are raw materials for synthesis, have biological toxicity.

Hemostats containing collagen as a single component, such as "Avitene" (Alcon) and "Helitene" (Duhamed) are non-blood products; however, since these agents are very expensive and are single-component preparations having no tissue adhering effect, the agents are used only as hemostats.

In addition, "Fibrin monomer-based tissue adhesive, US Patent 5464471, 1995", "Hemostatic and tissue adhesive, US Patent 5883078, 1999", "Fibrinogen/chitosan hemostatic agents, US Patent 5773033, 1998", "therapeutic fibrinogen compositions, US Patent 5605887, 1997", and the like have been introduced as other related art technologies. However, these have problems that since blood products such as fibrinogen, thrombin, blood coagulants, and aprotinin, bovine-derived proteins, and the like are used in adhesive hemostat products, there is a risk of contagion of particular diseases that can be induced at this time, and the manufactured products are highly expensive due to the excessive cost required for raw materials securement and storage, production processes, and the like.

Furthermore, FloSeal (Baxter) containing a gelatin matrix component is a most frequently used product and is a product that exhibits a hemostatic effect by adding a calcium chloride solution mixed with thrombin to a gelatin matrix. This can be used particularly for hemorrhages that are not effectively controllable or uncontrollable by ligature or general procedures during surgical operations in various regions including sites other than the eyes. However, since this product is separated into three major components, namely, thrombin, gelatin, and a calcium chloride solution, and solutions of each of these three components are not mixed at once but are mixed in two steps, the process is time-consuming. Therefore, during a surgical operation, when serious ligature and hemorrhage occur in an extremely urgent state, there is no time to mix the components according to the manual of such a complicated product, the operation that proceeds sequentially may threaten the life of an urgent bleeding patient, and this product has a problem of causing inconvenience to the user.
US 2012/021058 discloses a dry and stable hemostatic composition containing collagen granules coated with thrombin and stabilizers, in particular human serum albumin and mannitol. This document also discloses a kit comprising the dry and stable hemostatic composition in one container and a diluent in another container, so that combination of the hemostatic composition and the diluent forms a hydrogel.
EP2575775 discloses a dry and storage-stable hemostatic composition comprising a cross-linked gelatin, instead of collagen, a dry thrombin preparation and stabilizers, in particular human serum albumin, mannitol or sodium acetate. This document also discloses a kit comprising a first container comprising the hemostatic composition and a second container comprising a diluent. The hemostatic composition is to be contacted with the diluent before administration so as to obtain a hemostatic composition in hydrogel form.
Both these documents fail to disclose a hemostatic composition with collagen, thrombin and a stabilizer in powder form and structured as layers.

Since hemostatic products such as described above are applied to human beings, the highest safety criteria for the quality of the final product and components included therein, storage stability, and a simple method of use should be provided. Particularly, in the case of mixing two or more components and using the mixture, the mixture should be produced in a "ready-to-use" form and should be provided in an easily miscible state.

Accordingly, it is necessary to develop a hemostat that has very low toxicity, can be easily stored and handled, can be applied to urgent bleeding patients due to a simple method of use, and has excellent biodegradability.

### CITED REFERENCES

### Patent Reference

Patent Document 1: US Patent No. 5464471
Patent Document 2: US Patent No. 5773033
Patent Document 3: US Patent No. 5605887

### DISCLOSURE OF INVENTION

### PROBLEM TO BE SOLVED BY THE INVENTION

In order to solve problems such as described above, the inventors of the present invention developed a hemostatic composition including collagen, a stabilizer, and thrombin, and a container holding this hemostatic composition, and the inventors found that the hemostatic composition can be applied to urgent bleeding patients by a simple method of use, is not toxic, has no problem about blood infection, has a fast rate of biodegradation, and has an excellent hemostatic effect, thus completing the present invention.

Therefore, it is an object of the present invention to provide a hemostatic composition including collagen, a stabilizer, and thrombin, and a container holding this composition.

Furthermore, it is another object of the present invention to provide a kit including a first container filled with the above-described hemostatic composition and a second container filled with a diluent.

Moreover, it is still another object of the present invention to provide a hydrogel produced using the above-described hemostatic kit.

### MEANS FOR SOLVING PROBLEM

In order to achieve the above-described objects, according to an aspect of the present invention, there is provided a hemostatic composition in the form of a powder preparation, the composition including collagen, a stabilizer, and thrombin, wherein the collagen and the thrombin are disposed adjacently to each other.

The present invention provides a hemostatic composition including a first layer containing a collagen powder; a second layer containing a stabilizer powder, wherein the stabilizer is one or more selected from the group consisting of albumin, preferably human serum albumin, mannitol, sodium acetate C₂H₃NaO₂, sucrose,trehalose, sorbitol, and glycine, and a third layer containing a thrombin powder, the three layers being packed in a container in a layer-separated manner, wherein the first layer containing a collagen powder and the third layer containing a thrombin powder are disposed adjacently to each other, and the second layer containing a stabilizer powder is disposed at a position that is mixed first with the diluent.

The hemostatic composition may be disposed in the order of a stabilizer, thrombin, and collagen or in the order of a stabilizer, collagen, and thrombin, or the hemostatic composition may be disposed in the order of thrombin, collagen, and a stabilizer or in the order of collagen, thrombin, and a stabilizer.

The hemostatic composition may be disposed in the order of the second layer containing a stabilizer powder, the third layer containing a thrombin powder, and the first layer containing a collagen powder, or in the order of the second layer containing a stabilizer powder, the first layer containing a collagen powder, and the third layer containing a thrombin powder, or the hemostatic composition may be disposed in the order of the third layer containing a thrombin powder, the first layer containing a collagen powder, and the second layer containing a stabilizer powder, or in the order of the first layer containing a collagen powder, the third layer containing a thrombin powder, and the second layer containing a stabilizer powder.

With regard to the hemostatic composition, the collagen may be a crosslinked collagen.

With regard to the hemostatic composition, the crosslinked collagen may be produced by a production method including:
S1) a step of treating collagen with ethanol or methanol;
S2) a step of adding an acid to the collagen treated in the step S1) and producing a collagen solution at pH 2 to 4;
S3) a step of bringing the collagen solution produced in the step S2) to a neutral state and then centrifuging the collagen solution to produce an esterified collagen;
S4) a step of adding a crosslinking agent to the esterified collagen produced in the step S3) and producing a crosslinked collagen; and
S5) a step of dispersing the crosslinked collagen produced in the step S4) in purified water and then freeze-drying the dispersion.

With regard to the hemostatic composition, the molecular weight of the crosslinked collagen may be 100,000 to 1,000,000 Daltons.

The hemostatic composition may include 40% to 97% by weight of the collagen with respect to the total weight of the hemostatic composition.

With regard to the hemostatic composition, the stabilizer is one or more selected from the group consisting of albumin (human serum albumin), mannitol, sodium acetate (C₂H₃NaO₂), sucrose, trehalose, sorbitol, and glycine.

With regard to the hemostatic composition, the stabilizer may be mannitol.

The hemostatic composition may include 1% to 30% by weight of the stabilizer with respect to the total weight of the hemostatic composition.

The hemostatic composition may include 2% to 50% by weight of the thrombin with respect to the total weight of the hemostatic composition.

According to another aspect of the present invention, there is provided a container holding the hemostatic composition.

According to still another aspect of the present invention, there is provided a hemostatic kit including a first container filled with a hemostatic composition, the first container packed with a first layer containing a collagen powder, a second layer containing a stabilizer powder, selected from the group consisting of albumin, preferably human serum albumin, mannitol, sodium acetate C2H3NaO2, sucrose, trehalose, sorbitol, and glycine, and a third layer containing a thrombin powder in a layer-separated manner; and a second container filled with a diluent, wherein in the first container, the first layer containing a collagen powder and the third layer containing a thrombin powder are disposed adjacently to each other, and the second containing a stabilizer powder is disposed at a position that is mixed first with the diluent.

With regard to the hemostatic kit, the diluent may be one or more selected from the group consisting of purified water, a calcium chloride (CaCl₂) solution, a sodium chloride (NaCl) solution, human serum albumin, and a sodium acetate (C₂H₃NaO₂) solution.

With regard to the hemostatic kit, the diluent may be a calcium chloride solution, and the diluent may include 0.001% to 30% by weight of calcium chloride with respect to the total weight of the diluent.

With regard to the hemostatic kit, the first container and the second container in the hemostatic kit may be combined with each other, the hemostatic composition and the diluent may be mixed to form a hydrogel, and the average pore size of the hydrogel may be 50 µm to 200 µm.

With regard to the hemostatic kit, the pH of the hydrogel may be 6 to 8.

With regard to the hemostatic kit, the first container may be filled with a stabilizer, thrombin, and collagen in this order or with a stabilizer, collagen, and thrombin in this order, or the first container may be filled with thrombin, collagen, and a stabilizer in this order or with collagen, thrombin, and stabilizer in this order.

More specifically, the first container may be filled with a second layer containing a stabilizer powder, a third layer containing a thrombin powder, and a first layer containing a collagen powder, or with a second layer containing a stabilizer powder, a first layer containing a collagen powder, and a third layer containing a thrombin powder, or may be filled with a third layer containing a thrombin powder, a first layer containing a collagen powder, and a second layer containing a stabilizer powder, or with a first layer containing a collagen powder, a third layer containing a thrombin powder, and a second layer containing a stabilizer powder.

With regard to the hemostatic kit, the collagen may be a crosslinked collagen.

With regard to the hemostatic kit, the crosslinked collagen can be produced by a production method including:
S1) a step of treating collagen with ethanol or methanol;
S2) a step of adding an acid to the collagen treated in the step S1) and producing a collagen solution at pH 2 to 4;
S3) a step of bringing the collagen solution produced in the step S2) to a neutral state and then centrifuging the collagen solution to produce an esterified collagen;
S4) a step of adding a crosslinking agent to the esterified collagen produced in the step S3) to produce a crosslinked collagen; and
S5) a step of dispersing the crosslinked collagen produced in the step S4) in purified water and then freeze-drying the dispersion.

With regard to the hemostatic kit, the molecular weight of the crosslinked collagen may be 100,000 to 1,000,000 Daltons.

The hemostatic kit may include 40% to 97% by weight of the collagen with respect to the total weight of the hemostatic composition.

With regard to the hemostatic kit, the stabilizer is one or more selected from the group consisting of albumin (human serum albumin), mannitol, sodium acetate (C₂H₃NaO₂), sucrose, trehalose, sorbitol, and glycine.

With regard to the hemostatic kit, the stabilizer may be mannitol.

The hemostatic kit may include 1% to 30% by weight of the stabilizer with respect to the total weight of the hemostatic composition.

The hemostatic kit may include 2% to 50% by weight of the thrombin with respect to the total weight of the hemostatic composition.

### EFFECT OF THE INVENTION

The hemostatic composition of the present invention including collagen, a stabilizer, and thrombin has an excellent hemostatic effect.

Furthermore, the hemostatic composition of the present invention including collagen, a stabilizer, and thrombin can be easily stored by stabilizing the activity of thrombin and can be used quickly and conveniently because the composition can be mixed under low load.

The container holding the hemostatic composition of the present invention including collagen, a stabilizer, and thrombin can be used quickly and conveniently, is less toxic, does not have a problem with blood infection, has a high rate of biodegradation, expands with a high coefficient of expansion upon contact with blood, and therefore has an excellent hemostatic effect.

Accordingly, the hemostatic composition of the present invention and a container holding the same can be used as a hemostatic kit.

### BRIEF DESCRIPTION OF DRAWINGS

FIG. 1 is a graph obtained by measuring the force required upon mixing Example 1 and Comparative Example 1 with a calcium chloride solution, with respect to the number of times of mixing.
FIG. 2 is SEM images for hydrogels respectively produced by mixing Example 1 and Comparative Example 1 with a calcium chloride solution.
FIG. 3 is a graph obtained by measuring the respective coefficients of expansion of Example 1 and Comparative Example 1.
FIG. 4 is a graph obtained by measuring the respective amounts of change in area for Example 1 and Comparative Example 1.
FIG. 5 is a graph obtained by measuring the time required for hemostasis by treating nephrectomized rat models respectively with Example 1 and Comparative Example 1.
FIG. 6 is images showing the hemostatic capability after treating nephrectomized rat models respectively with Example 1 and Comparative Example 1.

### BEST MODE(S) FOR CARRYING OUT THE INVENTION

Examples of the present invention will be described in detail below. However, these Examples are suggested only for illustrative purposes and are not intended to limit the present invention. It should be noted that the present invention is defined only by the scope of the claims that will be described below.

In regard to the description of the present invention, when it is considered that specific description of related known configurations or functions may make the subject matter of the present invention rather unclear, detailed description thereof will be omitted.

In the description of the constituent elements of the present invention, terms such as first, second, A, B, (a), and (b) may be used. These terms are only for the purpose of distinguishing the constituent elements assigned with the term from other constituent elements, and the essence of the constituent elements and the sequence or order of the constituent elements will not be limited by these terms.

In the description of the present invention, the terms describing the degree or extent, such as "approximately", "substantially", and "about", will be used to refer to the manufacturing and material tolerances inherent in the meanings mentioned. To facilitate understanding of the invention, an exact or absolute number is used to prevent unauthorized exploitation by an unscrupulous infringer of the disclosure referred to.

The present invention provides a hemostatic composition including collagen, a stabilizer, and thrombin, in which the collagen and the thrombin are disposed adjacently to each other, as disclosed in claim 1.

Specifically, the hemostatic composition may be filled with a stabilizer, thrombin, and collagen in this order or with a stabilizer, collagen, and thrombin in this order, or the hemostatic composition may be filled with thrombin, collagen, and a stabilizer in this order or with collagen, thrombin, and stabilizer in this order. The hemostatic composition is used after being mixed with a diluent at the time of use, and the stabilizer is disposed at a position that is mixed first with the diluent. Accordingly, the components are disposed in the order of a stabilizer, thrombin, and collagen or in the order of a stabilizer, collagen, and thrombin.

The hemostatic composition of the present invention is in the form that various components in the form of powder preparations are laminated in layers. Specifically, the hemostatic composition of the present invention is a hemostatic composition including a first layer containing a collagen powder, a second layer containing a stabilizer powder, and a third layer containing a thrombin powder, the three layers being packed in a container in a layer-separated manner, wherein the first layer containing a collagen powder and the third layer containing a thrombin powder are disposed adjacently to each other, and the second layer containing a stabilizer powder is disposed at a position that is mixed first with a diluent. Accordingly, the hemostatic composition may have the layers disposed in the order of the second layer containing a stabilizer powder - the third layer containing a thrombin powder - the first layer containing a collagen powder or in the order of the second layer containing a stabilizer powder - the first layer containing a collagen powder - the third layer containing a thrombin powder, or the hemostatic composition may have the layers disposed in the order of the third layer containing a thrombin powder - the first layer containing a collagen powder - the second layer containing a stabilizer powder or in the order of the first layer containing a collagen powder - the third layer containing a thrombin powder - the second layer containing a stabilizer powder.

The above-described collagen is included in the hemostatic composition and can induce a hemostatic effect by absorbing blood at the bleeding site and attracting blood platelets. Furthermore, when collagen adheres to platelets, the collagen does not bind in a bare state but binds first to von Willebrand's factor (vWF), which is a substance acting like an adhesive, to promote binding with a GPIb/IX complex, which is a membrane glycoprotein present on the cell surface of platelets, and thus collagen can carry out primary hemostasis.

The collagen may be proteins obtained by treating tissues of various animals with an acid or an alkali, or treating the tissues with enzymes such as pepsin, and performing extraction.

The hemostatic composition may contain 40% to 97% by weight of collagen with respect to the total weight of the hemostatic composition. At this time, when the collagen is included at a proportion of less than 40% by weight, the hemostatic effect may be poor, and when the collagen is included at a proportion of more than 97% by weight, thrombin and the stabilizer are included at relatively smaller proportions so that the overall chemical stability of the hemostatic composition may be poor, or the hemostatic effect may be lowered.

Furthermore, the collagen may be a crosslinked collagen, and at this time, the molecular weight of the crosslinked collagen is preferably 100,000 to 1,000,000 Daltons but is not limited thereto. When the molecular weight of the crosslinked collagen is less than 100,000 Daltons, the blood absorption rate is fast; however, blood coagulation is slowed, which may deteriorate the hemostatic effect. When the molecular weight of the crosslinked collagen is more than 1,000,000 Daltons, the blood absorption power may be significantly poor.

The crosslinked collagen can be produced by a chemical crosslinking method, a physical crosslinking method, or a method combining these methods, and at this time, the collagen crosslinked by a chemical crosslinking method is a self-crosslinked collagen obtained using a crosslinking agent. On the other hand, the collagen crosslinked by a physical crosslinking method is a collagen crosslinked by a dry heat treatment, ultraviolet irradiation, or gamma-ray irradiation.

Here, the crosslinking agent may be any one or more crosslinking agents selected from the group consisting of formaldehyde, glutaraldehyde, carbodiimides (EDC), and polyepoxy compounds. The crosslinking agent is preferably a carbodiimide (EDC) but is not limited to this.

The crosslinked collagen has excellent mechanical strength, can exhibit a physical angiopressure effect, and can stop a large quantity of bleeding.

The crosslinked collagen may be a collagen obtained by crosslinking an esterified collagen.

According to an embodiment of the present invention, the crosslinked collagen can be produced by a production method including:
S1) a step of treating collagen with ethanol or methanol;
S2) a step of adding an acid to the collagen treated in the step S1) and producing a collagen solution at pH 2 to 4;
S3) a step of bringing the collagen solution produced in the step S2) to a neutral state and then centrifuging the collagen solution to produce an esterified collagen;
S4) a step of adding a crosslinking agent to the esterified collagen produced in the step S3) to produce a crosslinked collagen; and
S5) a step of dispersing the crosslinked collagen produced in the step S4) in purified water and then freeze-drying the dispersion.

The step S1) is a step of treating collagen with ethanol or methanol and is a step for facilitating the process of dissolving collagen in ethanol or methanol and mixing a crosslinking agent into the solution later. This step is a step for producing a collagen solution.

Before the step S1), a step of treating animal skin tissues with an acidic solution and extracting atelocollagen may be further included.

The step S2) is a step of adding an acid to the collagen treated in the step S1) and producing a collagen solution at pH 2 to 4 and is a step for adding an acid to the collagen solution and placing the collagen in a state of being completely dissolved in ethanol or methanol.

The step S3) is a step of bringing the collagen solution produced in the step S2) to a neutral state and then centrifuging the collagen solution to produce an esterified collagen and is a step of removing telopeptides, each of which has a non-helical structure composed of about 12 to 27 amino acid residues, from the two ends of a collagen molecule and esterifying the parts from which the telopeptides have been removed. Here, telopeptides are known as a main cause inducing an immune reaction when collagen is introduced into a living body, and accordingly, it is preferable to remove the telopeptides and use atelocollagen or an esterified collagen in order to avoid an immune reaction at the time of using collagen as a raw material for pharmaceutical products or the like.

The step S3) may be a step of treating the collagen solution that has been brought to a neutral state with pepsin enzyme to remove telopeptides, primarily producing an esterified collagen having an ester functional group, and then separating a high-purity esterified collagen using centrifugal separation.

At this time, it is preferable that the centrifugal separation is carried out at 1,000 rpm to 30,000 rpm for 5 minutes to 3 hours so that only the esterified collagen can be settled; however, there are no limitations.

Here, the method of separating the high-purity esterified collagen may include:
(a) a step of preparing a sample including atelocollagen, from which telopeptides have been removed, in a container;
(b) a step of allowing the sample including atelocollagen to flow from the container into a filtration module equipped with a filtering membrane and performing an ultrafiltration process of applying pressure to the filtration module and allowing the sample to be filtered by passing through the filtering membrane;
(c) a step of collecting an esterified collagen solution that has been filtered by passing through the filtering membrane in the ultrafiltration process and flows out from the filtration module;
(d) a step of measuring the flow rate of the esterified collagen solution that has been filtered by passing through the filtering membrane and determining the rate of ultrafiltration;
(e) a step of stopping the ultrafiltration process when the rate of ultrafiltration is lowered to or below a certain rate;
(f) a step of adding water to a residue of the sample that has not passed through the filtering membrane and returns to the container, subsequently allowing this mixture to flow into the filtration module equipped with a filtering membrane to perform a diafiltration process;
(g) a step of collecting the esterified collagen solution that has been filtered by passing through the filtering membrane in the diafiltration process and flows out from the filtration module; and
(h) a step of repeating the step (f) and the step (g).

In the step (b), a sample including the above-described atelocollagen can be caused to flow from the container into the filtration module equipped with a filtering membrane by a pumping action using a pump, and a pressure of about 10 to 30 psi can be applied to the filtration module.

In the step (e), when the rate of ultrafiltration is reduced to about 1 g/min or less, the ultrafiltration process can be terminated.

To the residue that is returned to the container in the step (f), purified water in the same amount as that of the solution filtered through the ultrafiltration process can be added.

It is preferable that the diafiltration process in the step (f) and step (g) is carried out at least five times.

Accordingly, a high-purity esterified collagen can be obtained while esterifying atelocollagen in the steps (a) to (h).

On the other hand, the step S4) is a step of producing a crosslinked collagen by adding a crosslinking agent to the esterified collagen produced in the step S3) and is a step for crosslinking the esterified collagen and providing a crosslinked collagen that can exhibit a physical angiopressure effect but does not cause an immune reaction.

Furthermore, the crosslinking agent for the step S4) may be any one or more crosslinking agents selected from the group consisting of formaldehyde, glutaraldehyde, carbodiimides (EDC), and polyepoxy compounds as described above, and detailed description will not be repeated here.

The step S5) is a step of dispersing the crosslinked collagen produced in the step S4) in purified water and then freeze-drying the dispersion and is a step for removing the crosslinking agent remaining in the crosslinked collagen and the collagen from which telopeptide has not been removed. Thus, in the step S5), the crosslinked collagen produced in the step S4) is dispersed in purified water, thereby a trace amount of the crosslinking agent and the collagen from which telopeptide has not been removed are dissolved in purified water, and these substances can be frozen while being dissolved in purified water through freeze-drying and removed. Furthermore, freeze-drying used in this case may be a conventional method that is used in the pertinent art.

The stabilizer is included in the hemostatic composition, stabilizes the activity of thrombin, and allows the composition to be rapidly mixed under low load at the time of mixing with a diluent. Therefore, as the stabilizer is included in the hemostatic composition, when collagen and thrombin having a strong hemostatic effect are mixed with a diluent, mixing can be achieved rapidly under low load, and retainment of the chemical stability and biological activity of the hemostatic composition can be secured by a non-active substance.

The stabilizer is one or more selected from the group consisting of albumin (human serum albumin), mannitol, sodium acetate (C₂H₃NaO₂), sucrose, trehalose, sorbitol, and glycine.

At this time, by incorporating one or more selected from the group consisting of albumin, mannitol, and sodium acetate as the stabilizer, the chemical stability and biological activity of thrombin can be retained.

Furthermore, by incorporating one or more selected from the group consisting of mannitol, sucrose, trehalose, sorbitol, and glycine as the stabilizer, prolongation of the hemostasis time can be suppressed, and the blood coagulation activity can be retained.

By incorporating one or more selected from the group consisting of mannitol, sucrose, trehalose, sorbitol, and glycine as the stabilizer, the composition can be mixed rapidly under low load.

The stabilizer is preferably mannitol but is not limited to this.

The above-described mannitol is white needle-like or column-like crystalline substance widely present in plants such as mushrooms and roots of pomegranate. Mannitol is highly soluble in water and may be used as a laxative or as a substitute for glycerol.

Particularly, mannitol significantly increases the rate of reconstitution of a dry powder, maintains the consistency of a reconstituted powder, reduces the moisture absorptivity of a dry powder, decreases the agglomeration strength between powder particles, and increases the flowability of a powder.

The hemostatic composition may include 1% to 30% by weight of the stabilizer with respect to the total weight of the hemostatic composition. At this time, when the stabilizer is included at a proportion of less than 1% by weight, it is difficult to retain the chemical stability and biological activity of collagen and thrombin, high load is required at the time of mixing with a diluent, and the mixing convenience may be deteriorated. When the stabilizer is included at a proportion of more than 30% by weight, the amounts of collagen and thrombin included may become relatively small so that the overall hemostatic effect of the hemostatic composition may be lowered.

Thrombin is included in the hemostatic composition and participates in blood coagulation as a powerful hemostatic factor to play the role as a catalyst in a reaction of hydrolyzing soluble fibrinogen in blood and converting the fibrinogen into insoluble fibrin. Furthermore, this thrombin can induce platelet activation in the process in which fibrin is formed by activation of blood coagulation factors, which is a secondary hemostasis process.

The hemostatic composition may include 2% to 50% by weight of thrombin with respect to the total weight of the hemostatic composition. At this time, when thrombin is included at a proportion of less than 2% by weight, the hemostatic effect may be very poor, and when thrombin is included at a proportion of more than 50% by weight, the amounts of collagen and the stabilizer included may become relatively small, the overall chemical stability of the hemostatic composition may be poor, or the hemostatic effect may be lowered.

The hemostatic composition is in the form of a powder preparation, and thus, it is preferable that the hemostatic composition in the form of a dried powder preparation is in the form of a powder preparation produced so as not to be easily degenerated into a liquid state by the external environment and moisture. As the hemostatic composition is in the form of a powder preparation, the hemostatic composition may have chemical stability due to the various components and can be easily retained in a state of not being mixed together. Furthermore, the hemostatic composition has an advantage of convenient handling and storage.

According to another embodiment of the present invention, a container holding the hemostatic composition can be provided.

At this time, since the container holding the hemostatic composition is in a state of being filled with all of collagen, a stabilizer, and thrombin, together having a hemostatic effect, the method of use is very simple, and the container may have an advantage of being applicable to urgent bleeding patients. Particularly, since the hemostatic composition can be applied directly to a bleeding site after being mixed only with a diluent in an urgent state where serious ligature and bleeding occur during a surgical operation and in an emergent state, the hemostatic composition has an advantage that the hemostatic composition is convenient for users and the method of use is very simple to the extent that even a non-professional technician can use the hemostatic composition.

According to another embodiment of the present invention, there is provided a hemostatic kit including a first container filled with a hemostatic composition including collagen, a stabilizer, and thrombin; and a second container filled with a diluent, wherein the first container is disposed adjacently to the collagen and thrombin.

Since the hemostatic kit uses the above-mentioned hemostatic composition, the collagen, the stabilizer, and the thrombin are also the same, and therefore, any further detailed description will not be repeated here.

The first container is filled with the hemostatic composition, collagen and thrombin are filled adjacently, and the order of disposition should not be changed by any external physical force. Furthermore, according to the order of disposition of the collagen, stabilizer, and thrombin filling in the first container, the flowability obtained after the components are mixed with a diluent may vary, and the coefficient of expansion and the rate of expansion may vary. Accordingly, the first container can be filled with a stabilizer, thrombin, and collagen in this order or with a stabilizer, collagen, and thrombin in this order, or can be filled with thrombin, collagen, and a stabilizer in this order or with collagen, thrombin, and a stabilizer in this order. The hemostatic composition can be used after being mixed with a diluent at the time of use, and in this case, the first container filled with the hemostatic composition and the second container filled with a diluent in the hemostatic kit are combined to mix the hemostatic composition and the diluent. The stabilizer is disposed at a position that is mixed first with the diluent Accordingly, with regard to the hemostatic composition in the first container, the hemostatic composition is disposed in the order of the stabilizer, thrombin, and collagen or in the order of the stabilizer, collagen, and thrombin, from the nozzle of the first container to be combined with the diluent.

The second container is filled with a diluent, and at this time, the diluent can be used as a solvent or a dispersing medium for dissolving or dispersing the hemostatic composition in the first container. For example, the diluent may be one or more selected from the group consisting of purified water, a calcium chloride (CaCl₂) solution, a sodium chloride (NaCl) solution, human serum albumin, and a sodium acetate (C₂H₃NaO₂) solution. The diluent is preferably a calcium chloride solution or a sodium chloride solution but is not limited to these.

When the diluent is a calcium chloride solution, the diluent may contain 0.001% to 30% by weight of calcium chloride with respect to the total weight of the diluent, and there are no particular limitations as long as the content is in a weight percent range in which mixing with the hemostatic composition is thoroughly achieved.

The shape and material of the first container or the second container are not particularly limited, and any shape and material conventionally used in the field of pharmaceutics and biotechnology can be used. For example, the shape of the first container or the second container is a syringe, and the material for the first container or the second container is a material that does not cause a chemical reaction with any one component selected from collagen, stabilizer, and thrombin.

On the other hand, in the hemostatic kit, the first container and the second container can be combined with each other, the hemostatic composition and the diluent are mixed, and thus a hydrogel can be formed.

In order for the first container and the second container to be combined together, the front end of the first container may include fastening protrusions, and the second container may include fastening grooves to be joined with the fastening protrusions present at the front end of the first container.

Furthermore, the hydrogel is porous, and therefore, when the hydrogel is used as a hemostat, the hydrogel has a high blood absorption ratio, the shape of the hydrogen is maintained even after absorbing blood so that the hemostatic effect is not lowered, and an excellent hemostatic effect can be exhibited.

The average pore size of the hydrogel may be 50 µm to 200 µm. When the average pore size is less than 50 µm, the blood absorption rate is decreased, and when the average pore size is more than 200 µm, the shape of the hydrogel cannot be maintained after absorbing blood, and the hemostatic effect may be deteriorated.

Furthermore, the hydrogel forming time is 5 seconds to 5 minutes, and serious ligature and hemorrhage in an urgent state can be prevented. As a hydrogel is formed within a short period of time, the user can easily use the hemostatic kit.

As the hydrogel acquires a pH of neutrality as a result of mixing of the hemostatic composition and the diluent, biocompatibility and biosafety can be secured when the hydrogel is applied to a living body. In addition, the hydrogel is less toxic and can be safe against infections. Accordingly, the pH of the hydrogel may be from 6 to 8.

According to another aspect of the present invention, not covered by the claims, there is provided use of the hemostatic composition for hemostasis at a bleeding site or for the treatment of a bleeding wound.

According to still another aspect, not covered by the claims, there is provided a hemostasis method including a step of treating or remedying an individual in need of hemostasis with the above-described hemostatic composition. The term "individual" as used in the present invention includes all animals, including human being, which have the occurrence of bleeding in some part of the body and require hemostasis. By treating or remedying the individual with the hemostatic composition of the present invention or applying or administering the hemostatic composition to the individual, hemostasis can be achieved effectively.

### EXAMPLES

Hereinafter, preferable Examples will be described in order to help understanding of the present invention.

### Production Example 1: Production of esterified collagen powder

An esterified collagen powder was produced by the following procedure.
(1) Collagen was added to ethanol, and then the mixture was stirred in a refrigerated state.
(2) The pH of the sample was adjusted to 6.7 ± 0.3 using 0.5 M HCl.
(3) The above-described solution was placed in a dialysis tubing, and dialysis was performed using purified water.
(4) The above-described solution was frozen and freeze-dried to obtain an esterified collagen powder.

### Production Example 2: Production of crosslinked collagen powder

A crosslinked collagen powder was produced by the following procedure.
(1) The esterified collagen produced in Production Example 1 was added to purified water and stirred.
(2) EDC (1-ethyl-3-(3-dimethylaminopropyl)-carbodiimide) was introduced into the esterified collagen solution that was sufficiently mixed.
(3) The mixture was thoroughly mixed so that EDC would sufficiently react with the esterified collagen.
(4) The mixture was left to stand for 2 to 3 days in a refrigerated state.
(5) The crosslinked collagen solution that had been crosslinked and solidified was added to purified water and dispersed therein.
(6) The crosslinked collagen solution was left to stand, and when layer separation occurred, only the crosslinked collagen was collected while the solution was discarded.
(7) The process of (6) was repeated 3 to 5 times to obtain only the crosslinked collagen.
(8) The crosslinked collagen was added to a Buffer (sodium phosphate/sodium chloride), and the mixture was left to stand for 1 to 2 days in a refrigerator to induce buffer neutralization.
(9) After the buffer neutralization, the crosslinked collagen was washed with purified water, and the collagen was frozen and freeze-dried.
(10) The sample after freeze-drying was ground in a blender.
(11) The powder obtained as described above was sieved through a sieve, and particles having a size of 200 to 355 µm were screened.

### Experimental Example 1: Examination of flowability

An Example was prepared as shown in the following Table 1, in order to examine the difference in flowability at the time of mixing the hemostatic composition with a calcium chloride solution as a diluent according to the order of disposition of mannitol as a stabilizer, thrombin, and collagen in the hemostatic composition. At this time, regarding mannitol and thrombin, powder-type manufactured products sold in the market were used, and the crosslinked collagen obtained from Production Example 2 was used.

**[Table 1]**

| **Example** | **Order of disposition** |
|---|---|
| Example 1 | Mannitol - thrombin - crosslinked collagen |
| Example 2 | Mannitol - crosslinked collagen - thrombin |
| Example 3 | Thrombin - crosslinked collagen - mannitol |
| Example 4 | Crosslinked collagen - thrombin - mannitol |
| Comparative Example 1 | Thrombin - mannitol - crosslinked collagen |

Regarding Examples 1 to 4 and Comparative Example 1 prepared as shown in Table 1 above, the force required at the time of mixing was measured by the following method, and the results of the measurement are presented in Table 2.
1) Two syringes were prepared, one syringe was packed with various components in the order of disposition according to Examples 1 to 4 and Comparative Example 1 from the nozzle of the syringe toward the inlet port, and the syringe was packed with 90% by weight of crosslinked collagen, 5% by weight of mannitol, and 5% by weight of thrombin with respect to the total weight of the components packed in the syringe. The other syringe was packed with a 10% calcium chloride solution.
2) The two syringes were connected (the ends of the cylinders were joined by fitting into each other).
3) The syringe (male type) packed with Example 1 or Comparative Example 1 was fixed (clamped) using a machine.
4) The piston of the syringe (female type) filled with the calcium chloride solution was pushed using a machine, and the calcium chloride solution was inserted into the syringe filled with Example 1 or Comparative Example 1. The force required at this time was measured, and the force was designated as N1.
5) The positions of the syringes were changed, and the empty syringe was fixed (clamped) using a machine.
6) The piston of the syringe (male type) filled with the mixed contents was pushed, and the mixed contents were inserted into the empty syringe. The force required at this time was measured, and the force was designated as N2.
7) The steps 5) and 6) were repeated, and the force required at the time of mixing was measured. Here, the required force (N3, N4, ..., N20) was measured every time the step 6) was carried out.

**[Table 2]**

| Number of mixing (times) | Maximum load (N) | |
|---|---|---|
| | Comparative Example 1 | Example 1 |
| N1 | 29.35 | 25.10 |
| N2 | 27.69 | 31.47 |
| N3 | 29.87 | 30.56 |
| N4 | 21.78 | 23.32 |
| N5 | 21.33 | 21.83 |
| N6 | 18.87 | 20.69 |
| N7 | 20.86 | 18.28 |
| N8 | 16.73 | 16.96 |
| N9 | 17.48 | 19.70 |
| N10 | 16.96 | 17.64 |
| N11 | 15.19 | 15.33 |
| N12 | 14.87 | 15.18 |
| N13 | 14.87 | 14.82 |
| N14 | 13.90 | 15.28 |
| N15 | 14.87 | 14.21 |
| N16 | 13.11 | 14.50 |
| N17 | 14.43 | 12.92 |
| N18 | 13.28 | 13.10 |
| N19 | 11.57 | 12.27 |
| N20 | 12.22 | 12.82 |
| Average | 17.96 | 18.30 |

As can be seen in the Table 2 above, it was found in Comparative Example 1 and Example 1 that the magnitudes of the force required for mixing, that is, the pressure occurring at the time of mixing, were similar. That is, Comparative Example 1 and Example 1 showed a tendency that the maximum load rapidly decreased from N4 identically in the two groups and substantially decreased until N20, and it was confirmed from this tendency that overall mixing of the contents was completed after N4, and the pressure occurring at the time of mixing gradually decreased and became constant. From these test results, it was verified that in the case of Comparative Example 1 packed with thrombin - mannitol - crosslinked collagen in this order as well as in the case of Example 1 packed with mannitol - thrombin - crosslinked collagen in this order, the pressure required for mixing was reduced, and the raw materials were effectively mixed.

On the other hand, in the case of Example 1 as well as in the case of Example 2 in which mannitol - crosslinked collagen - thrombin were disposed in this order, results similar to those of Example 1 were obtained, and in the case of Example 3 in which thrombin - crosslinked collagen - mannitol were disposed in this order and Example 4 in which crosslinked collagen - thrombin - mannitol were disposed in this order, it was verified that the magnitude of the pressure occurring at the time of mixing was somewhat large.

### Experimental Example 2: Examination of microstructure by SEM (Scanning Electron Microscope)

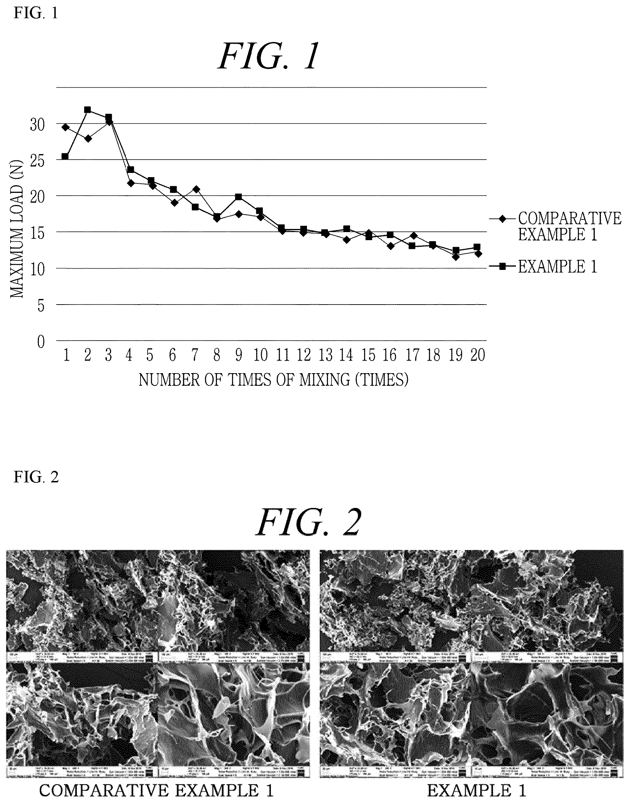

After carrying out the Experimental Example 1, images of the respective microstructures of Comparative Example 1 and Example 1, both of which had been mixed with the calcium chloride solution, were obtained using SEM. These images are shown in FIG. 2.

As can be seen in FIG. 2, in the case of Comparative Example 1 packed with thrombin - mannitol - crosslinked collagen in this order as well as in the case of Example 1 having mannitol - thrombin - crosslinked collagen disposed in this order, a fine and dense porous membrane structure can be identified. Accordingly, it was verified that in the case of being packed with thrombin - mannitol - collagen in this order as well as in the case of being packed with mannitol - thrombin - crosslinked collagen in this order, after the hemostatic composition was mixed with a calcium chloride solution, the hemostatic composition has a porous membrane structure that absorbs blood due to a uniform porous structure and causes the blood to agglomerate, thus assisting in hemostasis.

### Experimental Example 3: Examination of coefficient of expansion and amount of area change

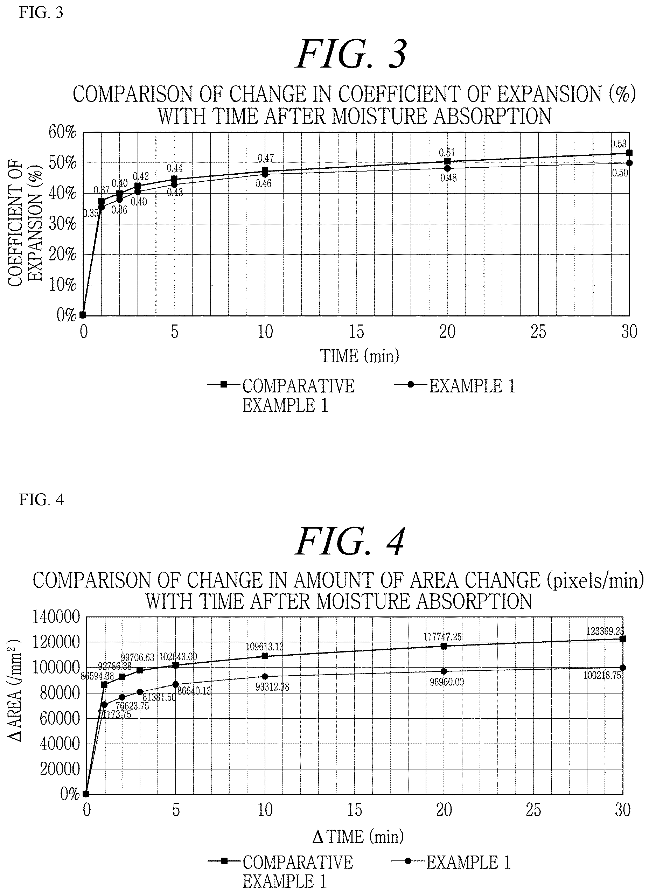

In order to examine the coefficient of expansion and the amount of area change exhibited by the hemostatic composition according to the present invention after mixing with a diluent, first, Comparative Example 1 and Example 1 were respectively prepared according to the modes described in Table 1 of Experimental Example 1. The particle areas of Comparative Example 1 and Example 1 thus prepared were respectively measured. Next, each of the samples was mixed with a calcium chloride solution, subsequently the particle area was measured at time intervals of 1, 2, 3, 5, 10, 20, and 30 minutes, and the area change ratio and the coefficient of expansion of the sample were calculated. These results are shown in FIG. 3 and FIG. 4. Expansion of coefficient (%) = [Area of moisture-absorbed powder per unit time - area of dry powder]/area of dry powder × 100

As can be seen from FIG. 3 and FIG. 4, it was confirmed that Comparative Example 1 and Example 1 rapidly expand within 1 minute from the beginning and continuously expand until 2, 3, 5, and 10 minutes; however, after 10 minutes, the amount of area change reaches a saturated state.

### Experimental Example 4: Degradability test

In order to measure the biocompatibility and degradability of the collagen included in the hemostatic composition according to the present invention and to examine whether the hemostatic composition has biocompatibility or has a risk of infection due to low degradability, a degradability test was carried out. First, the crosslinked collagens of Example 1 and Comparative Example 1 were prepared, and the respective sample weights were measured. Next, each sample was introduced into a micro-centrifuge tube, and the tube was filled with 1 to 2 mL of 1XPBS buffer containing 25 units/mL of collagenase. The tube containing the sample was introduced into a water bath at 37°C and was left to stand for 24 to 72 hours. After the passage of 24, 48, and 72 hours, the sample was taken out and freeze-dried, and then the weight was measured. The weights of the sample measured before and after the degradability test were used to calculate the residual weight ratio by the following calculation formula. The results are shown in Table 3. Residual weight ratio (%) = [Dry weight of sample after degradability test]/[dry weight of sample before degradability test] × 100

**[Table 3]**

| **Example** | **Residual weight ratio at different degradation times (%)** | | |
|---|---|---|---|
| | **After 24 hours** | **After 48 hours** | **After 72 hours** |
| Comparative Example 1 | 1.140 | 1.164 | 0.800 |
| Example 1 | 0.315 | 1.587 | 0.700 |

Regarding the crosslinked collagens of Comparative Example 1 and Example 1, it was confirmed that the samples were almost degraded by collagenase within 72 hours. Therefore, it was confirmed that even if thrombin and collagen were disposed adjacently to each other as in the case of the present invention, the crosslinked collagen had a rapid degradation capability under the same conditions, and thus it was confirmed that biodegradability was high and the risk of infection was very low.

### Experimental Example 5: Comparison of hemostasis performance in nephrectomized rat model

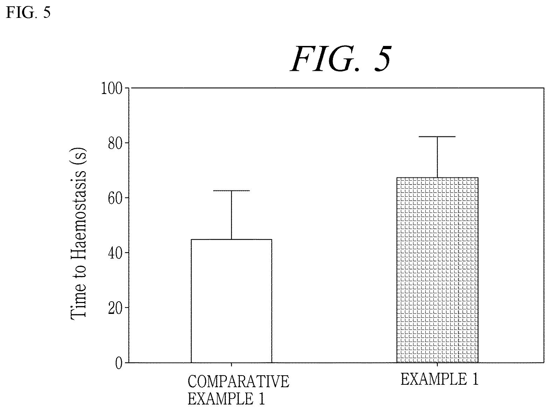

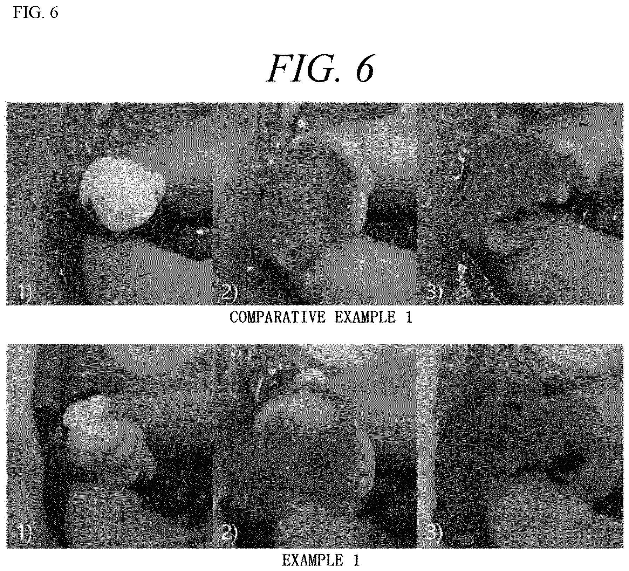

In order to examine the hemostatic capability of the hemostatic composition according to the present invention, Example 1 and Comparative Example 1 were prepared in the same manner as in Experimental Example 1 described above. The comparison of the hemostatic capability was carried out using a nephrectomized rat model. Kidneys were cut out to induce bleeding, subsequently Example 1 or Comparative Example 1 was applied thereto, and then angiopressure was attempted for 30 seconds to examine the presence or absence of bleeding. When bleeding occurred, additional angiopressure for 30 seconds was repeated. The time taken for hemostasis was measured, the results are shown in FIG. 5, and the process of hemostasis is shown in FIG. 6.

As can be seen from FIG. 5, the time taken for hemostasis was measured as about 68 seconds for Example 1 and about 45 seconds for Comparative Example 1. Accordingly, it was confirmed that even if thrombin and collagen were disposed adjacently as in the case of the present invention, the hemostatic composition had excellent hemostatic capability.

The foregoing description of the exemplary embodiments of the present invention has been provided for the purpose of illustration and description, and those having ordinary skill in the art to which the present invention is pertained will be able to understand that the present invention can be embodied in modified forms to the extent that maintains the essential characteristics of the present invention. Therefore, the disclosed Examples and Experimental Examples should be considered not from exhaustive viewpoints but from illustrative viewpoints. The scope of the present invention is disclosed not in the above-mentioned description but in the claims.

## Claims

1. A hemostatic composition including a first layer containing a collagen powder,
a second layer containing a stabilizer powder, wherein the stabilizer is one or more selected from the group consisting of albumin, preferably human serum albumin, mannitol, sodium acetate C₂H₃NaO₂, sucrose, trehalose, sorbitol, and glycine, and
a third layer containing a thrombin powder,
wherein the three layers being packed in a container in a layer-separated manner,
wherein the first layer containing a collagen powder and the third layer containing a thrombin powder are disposed adjacently to each other, and
the second layer containing a stabilizer powder is disposed at a position that is mixed first with a diluent.

2. The hemostatic composition according to claim 1,
wherein the hemostatic composition has the layers disposed in the order of the second layer containing a stabilizer powder - the third layer containing a thrombin powder - the first layer containing a collagen powder or in the order of the second layer containing a stabilizer powder - the first layer containing a collagen powder - the third layer containing a thrombin powder, or
the hemostatic composition has the layers disposed in the order of the third layer containing a thrombin powder - the first layer containing a collagen powder - the second layer containing a stabilizer powder or in the order of the first layer containing a collagen powder - the third layer containing a thrombin powder - the second layer containing a stabilizer powder.

3. The hemostatic composition according to claim 1, wherein the collagen is a crosslinked collagen having a molecular weight of 100,000 to 1,000,000 Daltons.

4. The hemostatic composition according to claim 1, wherein the hemostatic composition includes 40% to 97% by weight of the collagen, 1 to 30 wt% of the stabilizer, and 2 to 50 wt% of the thrombin with respect to the total weight of the hemostatic composition.

5. A container holding the hemostatic composition according to any one of claims 1 to 4.

6. A hemostatic kit comprising:
a first container filled with a hemostatic composition in the form of a powder preparation including a first layer containing a collagen powder, a second layer containing a stabilizer powder, and a third layer containing a thrombin powder, wherein the stabilizer is one or more selected from the group consisting of albumin, preferably human serum albumin, mannitol, sodium acetate C₂H₃NaO₂, sucrose, trehalose, sorbitol, and glycine; and
a second container filled with a diluent,
wherein the first container has the first layer containing a collagen powder and the third layer containing a thrombin powder disposed adjacently to each other, and
the second layer containing a stabilizer powder is disposed at a position that is mixed first with a diluent.

7. The hemostatic kit according to claim 6, wherein the diluent is one or more selected from the group consisting of purified water, a calcium chloride (CaCl₂) solution, a sodium chloride (NaCl) solution, human serum albumin, and a sodium acetate (C₂H₃NaO₂) solution.

8. The hemostatic kit according to claim 6,
wherein the first container and the second container in the hemostatic kit are combined with each other, the hemostatic composition and the diluent are mixed to form a hydrogel, and
the average pore size of the hydrogel is 50 *µ*m to 200 *µ*m.

9. The hemostatic kit according to claim 6,
wherein the first container is filled with the second layer containing a stabilizer powder, the third layer containing a thrombin powder, and the first layer containing a collagen powder in this order or with the second layer containing a stabilizer powder, the first layer containing a collagen powder, and the third layer containing a thrombin powder in this order, or
the first container is filled with the third layer containing a thrombin powder, the first layer containing a collagen powder, and the second layer containing a stabilizer powder in this order or with the first layer containing a collagen powder, the third layer containing a thrombin powder, and the second layer containing a stabilizer powder in this order.

10. The hemostatic kit according to claim 6, wherein the collagen is a crosslinked collagen having a molecular weight of 100,000 to 1,000,000 Daltons.

11. The hemostatic kit according to claim 6, wherein the hemostatic composition includes 40% to 97% by weight of the collagen, 1 to 30 wt% of the stabilizer, and 2 to 50 wt% of the thrombin with respect to the total weight of the hemostatic composition.

## Patentansprüche

1. Hämostatische Zusammensetzung umfassend eine erste Schicht, die ein Kollagenpulver enthält,
eine zweite Schicht, die ein Stabilisatorpulver enthält, worin der Stabilisator eines oder mehrere, das bzw. die aus der Gruppe ausgewählt ist bzw. sind, die aus Albumin, vorzugsweise Humanserumalbumin, Mannit, Natriumacetat C₂H₃NaO₂, Saccharose, Trehalose, Sorbit und Glycin besteht, und
eine dritte Schicht, die ein Thrombinpulver enthält,
worin die drei Schichten in einem Behälter in einer Schicht-getrennten Weise gepackt sind,
worin die erste Schicht, die ein Kollagenpulver enthält, und die dritte Schicht, die ein Thrombinpulver enthält, aneinander angrenzend angeordnet sind, und
die zweite Schicht, die ein Stabilisatorpulver enthält, an einer Stellung angeordnet ist, die zuerst mit einem Verdünnungsmittel gemischt ist.

2. Hämostatische Zusammensetzung nach Anspruch 1,
worin die hämostatische Zusammensetzung die Schichten hat, die in der Reihenfolge der zweiten Schicht, die ein Stabilisatorpulver enthält - der dritten Schicht, die ein Thrombinpulver enthält - der ersten Schicht, die ein Kollagenpulver enthält, angeordnet sind, oder in der Reihenfolge der zweiten Schicht, die ein Stabilisatorpulver enthält - der ersten Schicht, die ein Kollagenpulver enthält - der dritten Schicht, die ein Thrombinpulver enthält, angeordnet sind, oder
die hämostatische Zusammensetzung die Schichten hat, die in der Reihenfolge der dritten Schicht, die ein Thrombinpulver enthält - der ersten Schicht, die ein Kollagenpulver enthält - der zweiten Schicht, die ein Stabilisatorpulver enthält, angeordnet sind, oder in der Reihenfolge der ersten Schicht, die ein Kollagenpulver enthält - der dritten Schicht, die ein Thrombinpulver enthält - der zweiten Schicht, die ein Stabilisatorpulver enthält, angeordnet sind.

3. Hämostatische Zusammensetzung nach Anspruch 1, worin das Kollagen ein quervernetztes Kollagen ist, das ein Molekulargewicht von 100.000 bis 1.000.000 Dalton hat.

4. Hämostatische Zusammensetzung nach Anspruch 1, worin die hämostatische Zusammensetzung 40 Gew.-% bis 97 Gew.-% des Kollagens, 1 bis 30 Gew.-% des Stabilisators und 2 bis 50 Gew.-% des Thrombins in Bezug auf das Gesamtgewicht der hämostatischen Zusammensetzung umfasst.

5. Behälter, der die hämostatische Zusammensetzung nach einem der Ansprüche 1 bis 4 hält.

6. Hämostatisches Kit umfassend:
einen ersten Behälter, der mit einer hämostatischen Zusammensetzung in Form von einer Pulverzubereitung gefüllt ist, die eine erste Schicht, die ein Kollagenpulver enthält, eine zweite Schicht, die ein Stabilisatorpulver enthält, und eine dritte Schicht, die ein Thrombinpulver enthält, umfasst, worin der Stabilisator eines oder mehrere, das bzw. die aus der Gruppe ausgewählt ist bzw. sind, die aus Albumin, vorzugsweise Humanserumalbumin, Mannit, Natriumacetat C₂H₃NaO₂, Saccharose, Trehalose, Sorbit und Glycin besteht; und
einen zweiten Behälter, der mit einem Verdünnungsmittel gefüllt ist,
worin der erste Behälter die erste Schicht hat, die ein Kollagenpulver enthält, und die dritte Schicht, die ein Thrombinpulver enthält, die aneinander angrenzend angeordnet sind, und
die zweite Schicht, die ein Stabilisatorpulver enthält, an einer Stellung angeordnet ist, die zuerst mit einem Verdünnungsmittel gemischt ist.

7. Hämostatisches Kit nach Anspruch 6, worin das Verdünnungsmittel eines oder mehrere, das bzw. die aus der Gruppe ausgewählt ist bzw. sind, die aus gereinigtem Wasser, einer Kalziumchlorid (CaCl₂)-Lösung, einer Natriumchlorid (NaCl)-Lösung, Humanserumalbumin und einer Natriumacetat (C₂H₃NaO₂)-Lösung besteht.

8. Hämostatisches Kit nach Anspruch 6,
worin der erste Behälter und der zweite Behälter im hämostatischen Kit miteinander kombiniert sind, die hämostatische Zusammensetzung und das Verdünnungsmittel gemischt sind, um ein Hydrogel zu bilden, und
die durchschnittliche Porengröße des Hydrogels 50 µm bis 200 µm ist.

9. Hämostatisches Kit nach Anspruch 6,
worin der erste Behälter mit der zweiten Schicht, die ein Stabilisatorpulver enthält, der dritten Schicht, die ein Thrombinpulver enthält, und der ersten Schicht, die ein Kollagenpulver enthält, in dieser Reihenfolge gefüllt ist, oder mit der zweiten Schicht, die ein Stabilisatorpulver enthält, der ersten Schicht, die ein Kollagenpulver enthält, und der dritten Schicht, die ein Thrombinpulver enthält, in dieser Reihenfolge gefüllt ist, oder
der erste Behälter mit der dritten Schicht, die ein Thrombinpulver enthält, der ersten Schicht, die ein Kollagenpulver enthält, und der zweiten Schicht, die ein Stabilisatorpulver enthält, in dieser Reihenfolge gefüllt ist, oder mit der ersten Schicht, die ein Kollagenpulver enthält, der dritten Schicht, die ein Thrombinpulver enthält, und der zweiten Schicht, die ein Stabilisatorpulver enthält, in dieser Reihenfolge gefüllt ist.

10. Hämostatisches Kit nach Anspruch 6, worin das Kollagen ein quervernetztes Kollagen ist, das ein Molekulargewicht von 100.000 bis 1.000.000 Dalton hat.

11. Hämostatisches Kit nach Anspruch 6, worin die hämostatische Zusammensetzung 40 Gew.-% bis 97 Gew.-% des Kollagens, 1 bis 30 Gew.-% des Stabilisators und 2 bis 50 Gew.-% des Thrombins in Bezug auf das Gesamtgewicht der hämostatischen Zusammensetzung umfasst.

## Revendications

1. Composition hémostatique comprenant une première couche contenant une poudre de collagène,
une deuxième couche contenant une poudre de stabilisant, dans laquelle le stabilisant est un ou plusieurs éléments choisis dans le groupe constitué par l'albumine, de préférence la sérumalbumine humaine, le mannitol, l'acétate de sodium C₂H₃NaO₂, le saccharose, le tréhalose, le sorbitol et la glycine ; et
une troisième couche contenant une poudre de thrombine,
dans laquelle les trois couches sont conditionnées dans un récipient de manière à séparer les couches,
dans laquelle la première couche contenant une poudre de collagène et la troisième couche contenant une poudre de thrombine sont disposées de manière adjacente l'une à l'autre, et
la deuxième couche contenant une poudre de stabilisant est disposée à une position où elle est d'abord mélangée avec un diluant.

2. Composition hémostatique selon la revendication 1,
dans laquelle les couches de la composition hémostatique sont disposées dans l'ordre : deuxième couche contenant une poudre de stabilisant - troisième couche contenant une poudre de thrombine - première couche contenant une poudre de collagène ou dans l'ordre : deuxième couche contenant une poudre de stabilisant - première couche contenant une poudre de collagène - troisième couche contenant une poudre de thrombine, ou les couches de la composition hémostatique sont disposées dans l'ordre :
troisième couche contenant une poudre de thrombine - première couche contenant une poudre de collagène - deuxième couche contenant une poudre de stabilisant ou dans l'ordre : première couche contenant une poudre de collagène - troisième couche contenant une poudre de thrombine - deuxième couche contenant une poudre de stabilisant.

3. Composition hémostatique selon la revendication 1, dans laquelle le collagène est un collagène réticulé présentant un poids moléculaire de 100.000 à 1.000.000 Daltons.

4. Composition hémostatique selon la revendication 1, dans laquelle la composition hémostatique comprend 40% à 97% en poids de collagène, 1 à 30% en poids de stabilisant et 2 à 50% en poids de thrombine par rapport au poids total de la composition hémostatique.

5. Récipient contenant la composition hémostatique selon l'une quelconque des revendications 1 à 4.

6. Kit hémostatique comprenant :
un premier récipient rempli d'une composition hémostatique sous la forme d'une préparation en poudre comprenant une première couche contenant une poudre de collagène, une deuxième couche contenant une poudre de stabilisant et une troisième couche contenant une poudre de thrombine, dans lequel le stabilisant est un ou plusieurs éléments choisis dans le groupe constitué par l'albumine, de préférence la sérumalbumine humaine, le mannitol, l'acétate de sodium C₂H₃NaO₂, le saccharose, le tréhalose, le sorbitol et la glycine ; et
un second récipient rempli d'un diluant,
dans lequel le premier récipient comprend la première couche contenant une poudre de collagène et la troisième couche contenant une poudre de thrombine disposées de manière adjacente l'une à l'autre, et
la deuxième couche contenant une poudre de stabilisant est disposée à une position où elle est d'abord mélangée avec un diluant.

7. Kit hémostatique selon la revendication 6, dans lequel le diluant est un ou plusieurs éléments choisis dans le groupe constitué par l'eau purifiée, une solution de chlorure de calcium (CaCl₂), une solution de chlorure de sodium (NaCl), la sérumalbumine humaine et une solution d'acétate de sodium (C₂H₃NaO₂).

8. Kit hémostatique selon la revendication 6,
dans lequel le premier récipient et le second récipient du kit hémostatique sont combinés l'un à l'autre, la composition hémostatique et le diluant sont mélangés pour former un hydrogel, et
la taille moyenne des pores de l'hydrogel est de 50 µm à 200 µm.

9. Kit hémostatique selon la revendication 6,
dans lequel le premier récipient est rempli avec la deuxième couche contenant une poudre de stabilisant, la troisième couche contenant une poudre de thrombine, et la première couche contenant une poudre de collagène, dans cet ordre, ou avec la deuxième couche contenant une poudre de stabilisant, la première couche contenant une poudre de collagène, et la troisième couche contenant une poudre de thrombine, dans cet ordre, ou
le premier récipient est rempli avec la troisième couche contenant une poudre de thrombine, la première couche contenant une poudre de collagène, et la deuxième couche contenant une poudre de stabilisant, dans cet ordre, ou avec la première couche contenant une poudre de collagène, la troisième couche contenant une poudre de thrombine, et la deuxième couche contenant une poudre de stabilisant dans cet ordre.

10. Kit hémostatique selon la revendication 6, dans lequel le collagène est un collagène réticulé ayant un poids moléculaire de 100.000 à 1.000.000 Daltons.

11. Kit hémostatique selon la revendication 6, dans lequel la composition hémostatique comprend 40% à 97% en poids de collagène, 1 à 30% en poids de stabilisant et 2 à 50% en poids de thrombine par rapport au poids total de la composition hémostatique.
